(19) Europäisches Patentamt
European Patent Office
Office européen des brevets

(11) Veröffentlichungsnummer: **0 045 453**
**B1**

# (12) EUROPÄISCHE PATENTSCHRIFT

(45) Veröffentlichungstag der Patentschrift: **15.02.84**

(51) Int. Cl.³: **C 07 C 33/03**, C 11 B 9/00, A 23 L 1/221, A 61 K 7/46, C 07 C 29/40

(21) Anmeldenummer: **81105836.1**

(22) Anmeldetag: **23.07.81**

(54) **Neue Alkenole (I) und Verfahren zu deren Herstellung, Verwendung von (I) als Riech- und/oder Geschmackstoffe sowie Riech- und/oder Geschmackstoffkompositionen mit einem Gehalt an (I).**

(30) Priorität: **31.07.80 CH 5839/80**
**17.06.81 CH 3996/81**

(43) Veröffentlichungstag der Anmeldung: **10.02.82 Patentblatt 82/6**

(45) Bekanntmachung des Hinweises auf die Patenterteilung: **15.02.84 Patentblatt 84/7**

(84) Benannte Vertragsstaaten: **CH DE FR GB IT LI NL**

(56) Entgegenhaltungen:
**CH - A - 554 937**
**DE - A - 2 725 965**

**BERICHTE DER DEUTSCHEN CHEMISCHEN GESELLSCHAFT, 43. Jahrgang, Band III, 1910, Seiten 2330-2333, Nr. 370, Berlin, DE., E. BJELOUSS: "Über die Einwirkung der Grignardschen Verbindungen auf Methyl-äthyl-acrolein und über die Herstellung einiger Diolefine"**

(73) Patentinhaber: **L. GIVAUDAN & CIE Société Anonyme, CH-1214 Vernier-Genève (CH)**

(72) Erfinder: **Kaiser, Roman, Weidstrasse 6, CH-8610 Uster (CH)**
Erfinder: **Lamparsky, Dietmar, Dr., Sonnhalde 8, CH-8602 Wangen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

Neue Alkenole (I) und Verfahren zu deren Herstellung, Verwendung von (I) als Riech- und/oder Geschmackstoffe sowie Riech- und/oder Geschmackstoffkompositionen mit einem Gehalt an (I).

Die Erfindung betrifft neue Riech- und/oder Geschmackstoffe. Es handelt sich dabei um die Verbindungen der Formel

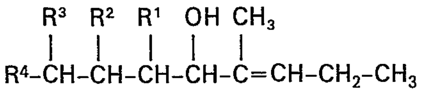
I

worin eines der Symbole $R^1$, $R^2$ und $R^3$ für Methyl oder Äthyl und die anderen für Wasserstoff stehen und $R^4$ Wasserstoff oder Methyl bedeutet, wobei im Falle $R^1 = R^2 =$ Wasserstoff und $R^3 =$ Methyl, $R^4$ Wasserstoff darstellt.

Die Formel soll demgemäss die sekundären $C_{10-12}$-Alkohole:

3,6-Dimethyl-6-nonen-5-ol [Ia],
4-Methyl-3-decen-5-ol [Ib],
4,6-Dimethyl-3-nonen-5-ol [Ic],
4-Methyl-3-nonen-5-ol [Id],
4,7-Dimethyl-3-octen-5-ol [Ie],
4,6-Dimethyl-3-octen-5-ol [If],
4-Methyl-6-äthyl-3-octen-5-ol [Ig],
4-Methyl-6-äthyl-3-nonen-5-ol [Ih],
4-Methyl-7-äthyl-3-nonen-5-ol [Ij],
4,8-Dimethyl-3-decen-4-ol [Ik],

in Form ihrer beiden möglichen Stereoisomeren (cis- bzw. trans-Konfiguration an der Doppelbindung) umfassen.

Die Erfindung betrifft ferner ein Verfahren zur Herstellung der Verbindungen der Formel I.

Dieses Verfahren ist dadurch gekennzeichnet, dass man 2-Methyl-2-pentenal mit einer Verbindung der Formel

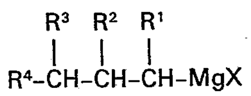
II

worin $R^1$ bis $R^4$ obige Bedeutung besitzen und X für Halogen steht, umsetzt.

Als Halogenide II kommen alle Halogenide in Frage, doch wird vorzugsweise das Bromid verwendet.

Die Umsetzung des Methylpentenals mit der Verbindung II erfolgt zweckmässigerweise nach den an sich bekannten Methoden der Grignardreaktion, siehe z.B. Organikum, Org. chem. Grundpraktikum, Nachdruck 15. Auflage, VEB deutscher Verlag der Wissenschaften, Berlin 1977, 617 seq. Man arbeitet also zweckmässigerweise in Diäthyläther als Lösungsmittel und bei Temperaturen von ca. 0–35°C.

Nach dem erfindungsgemässen Verfahren fällt I in Form eines Isomerengemischs an, in welchem die trans-Form von I stark überwiegt.

Aus wirtschaftlichen Gründen wird man insbesondere dieses Isomerengemisch verwenden.

Die Verbindungen I weisen besondere organoleptische Eigenschaften auf, auf Grund derer sie sich vorzüglich als Riech- und/oder Geschmackstoffe eignen.

Die Erfindung betrifft demgemäss auch die Verwendung der Verbindungen I als Riech- und/oder Geschmackstoffe.

Das 3,6-Dimethyl-6-nonen-5-ol Ia beispielsweise besitzt einen blumigen, fruchtigen und zugleich grün wirkenden Geruch mit warmem Unterton, wobei sich im Fond pudrige, an Schokolade erinnernde Nuancen feststellen lassen, währenddem das 4-Methyl-3-decen-5-ol Ib in erster Linie fruchtige und sehr natürlich wirkende Geruchsnoten besitzt, wobei besonders im Angeruch eine angenehm frisch-grüne und zugleich an Veilchen erinnernde Note auftritt. Man wird bei beiden Verbindungen u.a. an Grün-Noten erinnert, die man auch bei Margeriten und Tagetes beobachten kann. Diese Noten lassen sich bis jetzt nur schwer durch eine einzelne Substanz verwirklichen. Die z.B. als Inhaltsstoffe von Tagetes bekannten Tagetone bzw. Tagetenone sind ausserdem als konjugiert ungesättigte Ketone sehr instabil. Es ist festzuhalten, dass beispielsweise kein rosenartiger Geruch auftritt und sich Ia sowie Ib in ihrem gesamten Geruchsablauf sehr deutlich von bekannten Verbindungen ähnlicher Struktur abheben.

Generell wurde gefunden, dass die Alkohle der Formel I mit 11 Kohlenstoffatomen eine grünfruchtige Note mit Betonung des fruchtigen Charakters aufweisen, die entsprechenden Alkohole mit nur 10 Kohlenstoffatomen (Id–If) hingegen praktisch keinen Fruchtcharakter mehr aufweisen, sondern sich durch intensive frisch-grüne Geruchsnoten ohne blumige Nebennoten auszeichnen.

Die Verbindungen der Formel I eignen sich aufgrund ihrer natürlichen Geruchsnoten insbesondere zur Modifizierung von bekannten, z.B.

α) blumigen Kompositionen, in denen z.B. die Citrusnoten verstärkt zum Ausdruck kommen sollen (z.B. für Cologne-Typen u.ä., Extraits),
β) des weiteren aber auch von fruchtigen, z.B. Typ Himbeere (Extrait-Typen, Komposition der femininen Richtung), von
γ) Tabak- und Holzkompositionen, (Extrait-Typen der maskulinen Richtung) und schliesslich von
δ) Kompositionen mit grünen Noten, wo insbesondere eine erwünschte Abrundung und harmonisierende Effekte erzielt werden.

Geruchlich sehr interessante Effekte werden erzielt, wenn die Verbindung Ia und/oder Ib zusammen mit dem bekannten 2,6-Dimethyl-6-nonen-5-ol (III), also der Verbindung der Formel

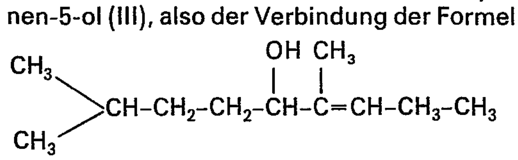
III

eingesetzt wird, wobei das Verhältnis von Ia und/oder Ib zu III in einem breiten Bereich variieren kann, also z.B. zwischen 1% Ia und/oder Ib und 99% III (oder umgekehrt) liegen kann.

Zwecks Herstellung der Gemische kann Ia und/ oder Ib III zugemischt werden, bzw. das für das erfindungsgemässe Verfahren benützte Ausgangsmaterial II kann beliebige Mengen an iso-Amylmagnesiumhalogenid enthalten.

Hervorzuheben ist insbesondere die durch die Verwendung der obigen Gemische erzielte aussergewöhnliche Diffusion der Riechstoffkompositionen. Aber auch erhöhte Frische und Natürlichkeit fallen auf.

In diesem Zusammenhang ist überraschend, dass III allein keinerlei organoleptisches Interesse beansprucht, so wird diese Verbindung beispielsweise von Bjelouss, in Ber. 43, 233 (1910) als von fadem Geruch beschrieben. Im Rahmen der vorliegenden Erfindung durchgeführte Versuche haben denn auch bestätigt, dass III in der Tat einen äusserst schwachen und nichtssagenden Eigengeruch aufweist. Auch die Bestimmung der Schwellenwerte (threshold value) bestätigt, dass es sich bei I um starke Riechstoffe handelt, bei III um einen schwachen.

Die Verbindungen I verbinden sich mit zahlreichen bekannten Riech- und/oder Geschmackstoffingredienzien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:

- Naturprodukte: Basilikumöl, Baummoos-Absolue, Beifussöl, Bergamotteöl, Cassisknospen-Absolue, Castoreum, Cedernholzöl, Ciste Labdanum, Civette, Corianderöl, Eichenmoos, Elemiöl, Fichtennadelöl, Galbanum, Geraniumöl, Jasmin-Absolue und sein synthetischer Ersatz, Jonquille-Absolue, Labdanum, Lavendelöl, Mandarinenöl, Mastix-Absolue, Palmarosaöl, Patchouliöl, Petitgrainöl, Paraguay, Sandelholzöl, Thymianöl, Weihrauch, Ylang-Ylang-Öl, Zitronenöl, etc.
- Alkohole: Citronellol, Geraniol, cis-3-Hexenol, Linalool, Phenyläthylalkohol, Rhodinol, Sandela® (3-Isocamphyl-5-cyclohexanol), etc.
- Aldehyde: α-Amylzimtaldehyd, Cyclamenaldehyd, Dodecanal, Heliotropin, α-Hexylzimtaldehyd, Hydroxycitronellal, 2,6,10-Trimethyl-undec-9-en-1-al (Adoxal®), Undecanal, ω-Undecylenaldehyd, etc.
- Ketone: Isoraldeine® (Isomethyl-α-jonon), α-Jonon, β-Jonon, 3-Prenylisocaranon, Vertofix® (= acetyliertes Cedernholzöl), etc.
- Ester: Amylsalicylat, Benzylacetat, Citronellylacetat, cis-3-Hexenylacetat, cis-3-Hexenylbenzoat, 1-Methyl-2-secbutylcyclohexylacetat, Methyldihydrojasmonat, Phenoxyäthylisobutyrat, Phenyläthyltiglat, Styrallylacetat, 2,3,6,6-Tetramethyl cyclohex-2-en-carbonsäure-äthylester, 3,6,6-Trimethyl-2-äthyl-cyclohexy-2-en-carbonsäure-äthylester, Vetivenylacetat, etc.
- Verschiedene: Cumarin, Eugenol, Isobutylchinolin, Limonen, p-Menthan-8-thiol-3-on, 1-Methylcyclododecyl-methyläther, γ-Nonalacton, γ-Undecalacton, Ambrettemoschus, Galaxolid® (1,3,4,6,7,8-Hexahydro-4,6,6,7,8,8-hexamethyl-cyclopenta-γ-2-benzopyran), Ketonmoschus, Musk 174® (12-Oxahexadecanolid), etc.

Die Verbindungen der Formel I bzw. Gemische von I mit III lassen sich in weiten Grenzen einsetzen, die beispielsweise von 0,1 (Detergentien) –50% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen, z.B. mit bis zu 40% neuartige Komplexe aufbauen kann. Die bevorzugten Konzentrationen bewegen sich zwischen 0,5 und 20%. Die mit I bzw. I + III hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I bzw. die Gemische I + III können demgemäss bei der Herstellung von Kompositionen und – wie obige Zusammenstellung zeigt – unter Verwendung einer breiten Palette bekannter Riechstoffe, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Als Geschmackstoffe können die Verbindungen I beispielsweise zur Erzeugung bzw. Verbesserung, Verstärkung, Steigerung oder Modifizierung von Frucht- (z.B. Melone, Pfirsich, Aprikose) oder Beerenaromen, insbesondere Himbeeraromen, oder von Schokoladearomen (Ia + III) in Nahrungsmitteln (Joghurt, Süsswaren, etc.); in Genussmitteln (Tee, Tabak, etc.) und Getränken (Limonaden etc.) verwendet werden.

Die ausgeprägten geschmacklichen Qualitäten der Verbindungen I ermöglichen die Verwendung in geringen Konzentrationen. Eine geeignete Dosierung umfasst beispielsweise den Bereich von 0,01–100 ppm, vorzugsweise von 0,1–20 ppm im Fertigprodukt, d.h. dem aromatisierten Nahrungsmittel, Genussmittel oder Getränk.

Bei der Aromatisierung von beispielsweise Tabak kann die Dosierung jedoch auch höher liegen und einen grösseren Bereich umfassen, beispielsweise den Bereich von 1 bis 1000 ppm, vorzugsweise 50–500 ppm.

Die Verbindungen können auf übliche Weise mit den für Geschmackstoffkompositionen verwendeten Bestandteilen vermischt bzw. solchen Aromen zugesetzt werden. Unter den erfindungsgemäss verwendeten Aromen werden Geschmackstoffkompositionen verstanden, die sich auf an sich bekannte Art verdünnen bzw. in essbaren Materialien verteilen lassen. Sie enthalten beispielsweise etwa 0,1–10, insbesondere 0,5–3 Gew.%. Sie können nach an sich bekannten Methoden in die üblichen Gebrauchsformen, wie Lösungen, Pasten oder Pulver übergeführt werden. Die Produkte können sprühgetrocknet, vakuumgetrocknet oder lyophilisiert werden.

Die bei der Herstellung solcher Aromen zweckmässigerweise verwendeten bekannten Aromastoffe sind entweder in der obigen Zusammenstellung bereits enthalten oder können leicht der Literatur entnommen werden, wie z.B. J. Merory, Food Flavorings, Composition, Manufacture and Use, Second Edition, The Avi Publishing Company, Inc., Westport, Conn. 1968, oder G. Fenaroli, Fenaroli's Handbook of Flavor Ingredients, Second Edition, Volume 2, CRC-Press, Inc. Cleveland, Ohio, 1975.

Für die Herstellung solcher üblicher Gebrauchsformen kommen beispielsweise folgende Trägermaterialien, Verdickungsmittel, Geschmackstoffverbesserer, Gewürze und Hilfsingredientien, etc. in Frage:

Gummi arabicum, Tragant, Salze oder Brauereihefe, Alginate, Carrageen oder ähnliche Absorbentien; Indole, Maltol, Dienale, Gewürzoleoresine, Raucharomen; Gewürznelken, Diacetyl, Natriumcitrat; Mononatriumglutamat, Dinatriuminodin-5'-monophosphat (IMP), Dinatriumguanosin-5-phosphat (GMP); oder spezielle Aromastoffe, Wasser, Äthanol, Propylenglykol, Glycerin.

Beispiel 1

3,6-Dimethyl-6-nonen-5-ol (Ia)

In einer für Grignard-Reaktionen üblichen Apparatur werden 0,72 g (= 29,8 mg-Atome) Magnesium in 3 ml absolutem Äther vorgelegt. Unter Rühren werden anschliessend 5,0 g (= 33,1 mMol) 1-Brom-2-methylbutan (90%ig) in 5 ml absolutem Äther so zugetropft, dass der Äther ständig siedet. Nach Beendigung der Zugabe des Bromids wird noch 30 Minuten bei Rückflusstemperatur gehalten, dann auf 10°C abgekühlt und einer Lösung von 2,92 g (= 29,8 mMol) 2-Methyl-2-pentenal in 3 ml absolutem Äther zugetropft. Die exotherme Reaktion lässt die Temperatur bis auf 25°C steigen. Nach einer weiteren Stunde Rückflussieren wird das Reaktionsprodukt mit gestossenem Eis und gesättigter Ammoniumchloridlösung in üblicher Weise aufgearbeitet. Die organische Schicht wird nach Abtrennung der wässrigen Phase mit gesättigter Kochsalzlösung gewaschen, anschliessend getrocknet und vom Lösungsmittel befreit. Das Rohprodukt (4,8 g) wird destilliert und liefert 1,74 g reines 3,6-Dimethyl-6-nonen-5-ol (Siedepunkt ca. 120°C/12 mmHg, $n_D^{20}$:1,4560), das folgende spektrale Daten besitzt:

IR (flüss. Film): 3350, 2960, 2928, 2876, 1670, 1462, 1378, 1004, 856 $cm^{-1}$.

NMR (360 MHz):0,88 + 0,89 (d+t, 6H), 0,965 (t, 3H); 1,59 (s, 3H); 2.015 (m, 2H), 4,08 (m, 1H); 5,38 (t, 1H)

MS (70 eV): m/e =170(3), 155(2), 141(12), 99(78), 81(27), 71(23), 55(28), 43(100).

Beispiel 2

4-Methyl-3-decen-5-ol (Ib)

In einer für Grignard-Reaktionen üblichen Apparatur werden 2,4 g (= 0,1 g-Atome) Magnesium in 50 ml absolutem Äther vorgelegt. Unter Rühren und Schutzgasatmosphäre (Stickstoff) werden anschliessend 15,0 g (= 0,1 Mol) n-Amylbromid in 50 ml absolutem Äther so zugetropft, dass der Äther nach Anspringen der Reaktion ständig schwach siedet. Nach Beendigung der Zugabe wird noch 30 Minuten bei Rückflusstemperatur gehalten, dann auf 10°C abgekühlt und eine Lösung von 7,85 g (= 0,08 Mol) 2-Methyl-2-pentenal in 20 ml absolutem Äther zugetropft. Zwecks Beendigung der Reaktion wird 12 Stunden bei Raumtemperatur weitergerührt. Nach Zersetzung des Grignard-Komplexes mit gesättigter Ammoniumchloridlösung und Eis wird die überstehende ätherische Lösung mit gesättigter Kochsalzlösung gewaschen und anschliessend getrocknet. Nach Abdampfen des Lösungsmittels verbleiben 13,6 g Rohprodukt, die fraktioniert destilliert werden. Man erhält so 8,9 g reines 4-Methyl-3-decen-5-ol vom Siedepunkt 103°C/12 mmHg, $n_D^{20}$: 1,4499.

Spektrale Daten:

IR: 3340, 2958 + 2924, 2888 + 1858, 1670, 1460, 1024, 854 $cm^{-1}$

NMR (360 MHz): 0,89 (t, 3H); 0,965 (t, 3H); 1,595 (s, 3H); 2,03 (m, 2H); 3,96 (t, 1H), 5,36 (t, 3H)

MS: m/e = 170 ($M^+$, 6), 155(1), 141(18), 128(2), 109(2), 99(100), 81(19), 71(15), 55(19), 43(42).

Beispiel 3

2,6-Dimethyl-6-nonen-5-ol ((III) + wenig Ib)

In 500 ml absolutem Äther werden 69,5 g (= 2,9 g-Atome) Magnesium vorgelegt. Anschliessend wird die Lösung von 438 g Isoamylbromid, das gemäss Gaschromatogramm ca. 1,5% n-Amylbromid enthält, in 1,2 l absolutem Äther so zugetropft, dass die exotherme Reaktion den Äther ständig beim Siedepunkt hält. Nach Beendigung der Zugabe wird noch 30 Minuten bei Rückflusstemperatur gehalten. Die Grignardlösung wird alsdann auf 10°C abgekühlt. Dann werden 236,5 g (= 2,41 Mol) 2-Methyl-2-pentenal in 600 ml absolutem Äther innert 40 Minuten zugetropft, die Temperatur liegt so dauernd zwischen 10 und 20°C. Zwecks Beendigung der Reaktion wird noch 1 Stunde bei Rückflusstemperatur gehalten. Das Reaktionsgemisch wird anschliessend auf Eis gegeben, mit wässriger Salzsäure zersetzt, die ätherische Lösung mit Soda und gesättigter Kochsalzlösung neutral gewaschen und hierauf getrocknet. Das nach Abdestillieren des Lösungsmittels verbleibende Rohprodukt (480 g) wird über eine Widmer-Kolonne fraktioniert destilliert und ergibt 312 g 2,6-Dimethyl-6-nonen-5-ol (III) vom Siedepunkt 62°/0,05 mmHg, $n_D^{25}$: 1,4479, das gemäss Gaschromatogramm (Carbowax, 130°) 1,5% 4-Methyl-3-decen-5-ol enthält.

Spektrale Daten (III):
IR: 3350, 2956 + 2930, 2866, 1670, 1468, 1386, 1368, 1012, 856 $cm^{-1}$
NMR (60 MHz): 0,88 + 0,90 (zusammenfallend, 9H); 1,59 (s, 3H); 2,02 (t, 2H); 3,93 (t, 1H); 5,33 (t, 1H)
MS: m/e = 170(7), 141(16), 123(4), 99(100), 81(34), 71(19), 55(36), 43(91).

Beispiel 4
1:1-Gemisch von 2,6-Dimethyl-6-nonen-5-ol (III) mit 4-Methyl-3-decen-5-ol (Ib)

Analog Beispiel 1 bis 3 wird zu 1,6 g Magnesium in 20 ml absolutem Äther die Lösung eines Gemisches von 5,0 g n-Amylbromid und 5,0 g Isoamylbromid in 40 ml absolutem Äther so zugetropft, dass der Äther ständig siedet. Nach beendeter Zugabe lässt man noch 30 Minuten bei der Rückflusstemperatur des Äthers nachreagieren und arbeitet dann wie in Beispiel 1-3 beschrieben auf. Es werden 8,4 g Rohprodukt erhalten, die nach Destillation im Vakuum 7,7 g eines Gemisches liefern, das gemäss Gaschromatogramm 54% 2,6-Dimethyl-6-nonen-5-ol und 46% 4-Methyl-3-decen-5-ol enthält (Siedepunkt des Gemisches 97-100°C/12 mmHg).

Beispiel 5
4,6-Dimethyl-3-nonen-5-ol

In einer für Grignard-Reaktionen üblichen Apparatur werden 28,3 g (1,18 g-Atome) Magnesium in 200 ml Äther vorgelegt. Unter Rühren und Schutzgasatmosphäre ($N_2$) werden anschliessend 178,1 g (1,18 Mol) 2-Brompentan in 500 ml absolutem Äther so zugetropft, dass der Äther nach Anspringen der Reaktion ständig schwach siedet. Nach beendeter Zugabe wird noch 30 Minuten bei Rückflusstemperatur gehalten, dann auf 10°C abgekühlt und eine Lösung von 96,1 g (0,98 Mol) 2-Methyl-2-pentenal in 300 ml Äther während 30 Minuten so zugetropft, dass die Reaktionstemperatur ständig zwischen 10° und 20° liegt. Zwecks Beendigung der Reaktion wird noch eine Stunde rückflussiert, dann der Grignard-Komplex mit gesättigter Ammoniumchloridlösung und Eis zersetzt, die überstehende ätherische Lösung mit gesättigter Kochsalzlösung gewaschen und anschliessend getrocknet. Nach Abdampfen des Lösungsmittels verbleiben 176 g Rohprodukt, die fraktioniert destilliert werden. Man erhält so 119 g (71,4%) olfaktisch gutes 4,6-Dimethyl-3-nonen-5-ol vom Siedepunkt 92°C/12 mm Hg.

Spektrale Daten:
IR: 3380, 2958, 2924, 2865, 1670, 1460, 1378, 1300, 1005, 854.
NMR: 0,70-1,20 (2t und 1d, gegenseitig überlagert, 9H); 1,58 (s, 3H), 2,02 (m, 2H); 3,67 (m, 1H); 5,34 (t, J~6,5, 1H).
MS: 170 ($M^+$, 2), 141(2), 128(3), 123(1), 109(1), 99(100), 81(25), 71(12), 55(11), 43(72).
Geruch: grün (Liguster), fruchtig-würzig, fettig, kakaoartig.

Beispiel 6
4-Methyl-3-nonen-5-ol

Analog Beispiel 5 wird das Grignardreagens [29,1 g (1,2 g-Atom) Magnesium in 300 ml Äther und 164 g (1,2 Mol) Butylbromid in 500 ml Äther] mit 98,15 g (1 Mol) 2-Methyl-2-pentenal in 200 ml Äther umgesetzt. Die fraktionierte Destillation des Rohproduktes (195,2 g) über eine 20 cm-Widmerkolonne ergibt 124,2 g (79,5%) olfaktisch gutes 4-Methyl-3-nonen-5-ol vom Siedepunkt 89-90°/12 mm Hg.

Spektrale Daten:
IR: 3350, 3958, 3925, 2870, 2858, 1670, 1460, 1380, 1305, 1110, 1050, 1002, 855.
NMR: 0,80-1,20 (2t, 6H); 1,60 (s, 3H); 2,02 (m, 2H); 3,98 (t, J~6,5, 1H); 5,36 (t, J~6,5, 1H).
MS: 156 ($M^+$, 2), 127(8), 114(2), 99(36), 81(16), 71(12), 57(12), 55(16), 43(100), 41(25).
Geruch: fettig, grün, diffusiv («montant»).

Beispiel 7
4,7-Dimethyl-3-octen-5-ol

Analog Beispiel 5 wird das Grignard-Reagens, welches durch Reaktion von 11,8 g (0,49 g-Atome) Magnesium in 50 ml Äther und 67,13 g (0,49 Mol) Isobutylbromid in 250 ml Äther erhalten wurde, mit 40,0 g (0,41 Mol) 2-Methyl-2-pentenal in 100 ml Äther umgesetzt. Die fraktionierte Destillation des Rohproduktes (94 g) über eine 20 cm-Widmerkolonne ergibt 44,4 g (69,4%) olfaktisch gutes 4,7-Dimethyl-3-octen-5-ol vom Siedepunkt 47-48°/0,04 mm Hg.

Spektrale Daten:
IR: 3350, 2958, 2930, 2865, 1670, 1468, 1383, 1367, 1305, 1050, 1000, 856.
NMR: 0,80-1,20 (1d + 1t, 9H); 1,60 (s, 3H); 2,02 (m, 2H); 4,08 (t, J~6,5, 1H); 5,37 (t, J~6,5, 1H).
MS: 156 ($M^+$, 9); 127(24), 114(16), 109(6), 99(100), 81(32), 71(26), 55(19), 43(80), 41(43).
Geruch: grün mit erdig-muffiger Nebennote.

Beispiel 8
4,6-Dimethyl-3-octen-5-ol

Analog Beispiel 5 wird das Grignard-Reagens, welches durch Reaktion von 18,7 g (0,77 g-Atom) Magnesium in 100 ml Äther und 105,4 g (0,77 Mol) 2-Brombutan in 300 ml Äther erhalten wurde, mit 62,72 g (0,64 Mol) 2-Methyl-2-pentenal in 200 ml Äther umgesetzt. Die fraktionierte Destillation des Rohproduktes (98,8 g) über eine 20 cm-Widmerkolonne ergibt 68,4 g (68,5%) olfaktisch gutes 4,6-Dimethyl-3-octen-5-ol vom Siedepunkt 83°/12 mm Hg.

Spektrale Daten:
IR: 3400, 2960, 2930, 2870, 1670, 1460, 1378, 1300, 1035, 1000, 858.
NMR: 0,70-1,10 (2t + 1d, gegenseitig überlagert, 9H); 1,58 (s, 3H); 2,02 (m, 2H); 3,68 (m, 1H); 5,37 (t, J~6,5, 1H).
MS: 156 ($M^+$, 1), 109(1), 99(54), 81(20), 71(10), 57(12), 55(15), 43(100), 41(22), 39(7).
Geruch: frisch, grün, krautig.

Beispiel 9

4-Methyl-6-äthyl-3-octen-5-ol

Analog Beispiel 5 wird das Grignard-Reagens, welches durch Reaktion von 29,4 g (1,21 g-Atom) Magnesium in 200 ml Äther und 183 g (1,21 Mol) 3-Brom-pentan in 400 ml Äther erhalten wird, mit 98,0 g (1,0 Mol) 2-Methyl-2-pentenal umgesetzt. Die fraktionierte Destillation des Rohproduktes (159,3 g) über eine 20 cm-Widmerkolonne ergibt 95,5 g (56,2%) olfaktisch gutes 4-Methyl-6-äthyl-3-octen-5-ol vom Schmelzpunkt 92–93°/12 mm Hg.

Spektrale Daten:
IR: 3400, 2960, 2930, 2870, 1670, 1460, 1378, 1300, 1040, 1010, 910, 855.
NMR: 0,70–1,10 (3t, gegenseitig überlagert, 9H); 1,60 (s, 3H); 2,08 (m, 2H); 3,80 (m, 1H); 5,33 (t, J~6,5, 1H);
MS: 170 ($M^+$, 1) 141(0,5); 99(71), 81(24); 71(18); 57(12), 55(22), 43(100), 41(28).
Geruch: fruchtig, kakao-artig, Aspekte von Maraschino.

Beispiel 10

4-Methyl-7-äthyl-3-nonen-5-ol

Analog Beispiel 5 wird das Grignardreagens, welches durch Reaktion von 11,4 g (0,47 g-Atom) Magnesium in 100 ml Äther und 77,6 g (0,47 Mol) 1-Brom-2-äthylbutan in 400 ml Äther erhalten wurde, mit 38,4 g (0,39 Mol) 2-Methyl-2-pentenal umgesetzt. Die fraktionierte Destillation des Rohproduktes (79,8 g) über eine 15 cm-Widmerkolonne ergibt 29,9 g (40,3%) olfaktisch gutes 4-Methyl-7-äthyl-3-nonen-5-ol vom Siedepunkt 105–106°/12 mm Hg.

Spektrale Daten:
IR: 3350, 2955, 2920, 2865, 1670, 1460, 1378, 1300, 1002, 853.
NMR: 0,70–1,10 (3t, gegenseitig überlagert, 9H); 1,60 (s, 3H); 2,05 (m, 2H); 4,10 (m, 1H); 5,38 (t, J~6,5, 1H).
Geruch: fruchtig, grün, blumig.

In den folgenden Formulierungsbeispielen steht A für ein Gemisch folgender Zusammensetzung: 98% III, 1,5% Ib und 0,5% Ia.

Beispiel 11

Parfümerie-Base Richtung modernes Cologne

| | Gewichtsteile |
|---|---|
| Myrascone® (2-Äthyl-3,6,6-trimethyl-2-cyclohexen-1-carbonsäureäthylester) | 80 |
| Galaxolide 50® IFF (50%ig) | 60 |
| Hydroxycitronellal | 60 |
| Madrox® (1-Methyl-1-methoxycyclododecan) | 60 |
| Sandela® (3-Isocamphyl-5-cyclohexanol) | 60 |
| Bergamotteöl | 60 |
| Fichtennadelöl | 30 |
| Keton-Moschus | 40 |
| Givescone® (2-Äthyl-6,6-dimethyl-2-cyclohexen-1-carbonsäureäthylester) | 20 |
| 3-Prenyl-isocaranon | 20 |
| Petitgrainöl Paraguay | 15 |
| p-Menthan-8-thiol-3-on | 5 |
| Baummoos Absolue | 5 |
| Propylenglykol | 450 |
| | 965 |

Ein Zusatz von 35 Teilen des neuen Gemischs A bringt diesem Cologne sehr viel mehr Leben, mehr Volumen; die Komposition wirkt kräftig fruchtig-würzig und zugleich ausgesprochen charmant, bezaubernd.

Beispiel 12

Parfümerie-Base in Richtung Calèche®

| | Gewichtsteile |
|---|---|
| Hydroxycitronellal | 250 |
| Vetivenylacetat | 100 |
| Bergamotteöl | 100 |
| Sandela® Giv | 100 |
| Phenyläthylalkohol | 60 |
| Isoraldeine®Giv (Isomethyl-α-jonon) | 60 |
| Jasmin synth. | 50 |
| Rhodinol | 50 |
| Keton-Moschus | 30 |
| Ylangöl | 20 |
| Dodecanal (10% in DPG) | 20 |
| Cumarin | 10 |
| Undecanal (10% in DPG) | 10 |
| Dipropylenglykol (DPG) | 80 |
| | 950 |

Der Zusatz von 50 Gewichtsteilen des neuen Substanzgemisches A zu obiger Komposition ruft in deren Kopfnote eine grössere Weichheit hervor und bewirkt eine angenehm fruchtig-blumige Nuancierung.

Beispiel 13

Parfürmerie-Base in Richtung Cabochard®

| | Gewichtsteile |
|---|---|
| Isoraldeine | 200 |
| Ambrette-Moschus | 100 |
| Phenyläthylalkohol | 100 |
| Bergamotteöl | 100 |
| Baummoos | 50 |
| Vetivenylacetat | 50 |
| Jasmin synth. | 50 |
| Patchouliöl | 40 |
| Rhodinol | 40 |
| Eugenol | 40 |
| Sandela®Giv | 40 |
| α-Hexylzimtaldehyd | 40 |
| Madrox® | 30 |
| Civette synth. (10% in Dipropylenglykol [DPG]) | 20 |
| Styrallylacetat | 20 |
| Castoreum synth. | 2 |

| | Gewichtsteile |
|---|---|
| Isobutylchinolin (10% in DPG) | 10 |
| Hydroxycitronellal | 50 |
| Undecylenaldehyd (10% in DPG) | 10 |
| Zitronenöl | 5 |
| γ-Undecalacton | 2 |
| Labdanum-Resinoid | 1 |
| | 1000 |

Fügt man zu obiger Komposition 100 Gewichtsteile des neuen Gemisches A, so wirkt die neue Komposition durch ihre blumig-fruchtige Tendenz sehr viel interessanter und besitzt eindeutig mehr Diffusion. Im Fond kommt eine grössere Süsse zur Geltung, die die etwas trockene Fondnote der Grundkomposition wesentlich verbessert.

Beispiel 14
Parfümerie-Base in Richtung grün-blumig

| | Gewichtsteile |
|---|---|
| Hydroxycitronellal | 250 |
| Methyldihydrojasmonat | 250 |
| Propylenglykol | 200 |
| Bergamotteöl | 100 |
| Citronellol | 50 |
| p-Menthan-8-thiol-3-on 1‰ | 10 |
| Mandarinenöl | 10 |
| Galbanumöl | 10 |
| Jasmin synth. | 10 |
| Palmarosaöl | 10 |
| Mastix-Absolue | 5 |
| Geraniumöl Bourbon | 5 |
| Cyclamenaldehyd | 5 |
| Corianderöl | 5 |
| Phenoxyäthylisobutyrat | 5 |
| cis-3-Hexenol (10% in Propylenglykol) | 5 |
| Basilikumöl | 3 |
| Cassis-Knospenöl (absolut) | 2 |
| | 935 |

Gibt man zu obiger grün-blumigen Base 65 Teile der neuen Substanz Ia, so tritt die Muguet- und Flieder-Note viel deutlicher hervor. Die Komposition wirkt nach Zusatz von Ia äusserst harmonisch.

Beispiel 15
Parfümerie-Komposition in Richtung Fougère

| | Gewichtsteile |
|---|---|
| Lavendelöl | 210 |
| Amylsalicylat | 200 |
| Baummoos-Absolue (50% in Dipropylenglykol) | 100 |
| Citronellol | 100 |
| Geraniol | 80 |
| Ambrette-Moschus | 80 |
| Bergamotteöl | 80 |
| α-Jonon | 80 |
| α-Amylzimtaldehyd | 25 |
| Eugenol | 20 |
| Metambrate® Giv (1-Acetoxy-1-methyl-2-sec.butyl-cyclohexan) | 25 |
| | 1000 |

Setzt man obiger Base 20% des neuen Gemisches A zu, so wird zwar die Lavendelnote gedämpft, die Veilchen-Kopfnote wirkt jedoch als sehr interessantes neues Fougère-Element. Der Fond wirkt sehr viel süsser und blumiger.

Beispiel 16
Parfümerie-Komposition in Richtung Chypre

| | Gewichtsteile |
|---|---|
| Madrox® Giv | 200 |
| Bergamotteöl | 150 |
| Hydroxycitronellal | 100 |
| Citronellol | 80 |
| Petitgrainöl | 60 |
| Musk 174® Naarden | 60 |
| Corianderöl | 40 |
| Galbanumöl | 40 |
| Zedernholzöl | 40 |
| Patchouliöl | 40 |
| Zitronenöl | 40 |
| Elemiöl | 10 |
| Eichenmoos | 25 |
| Fichtennadelöl Pumillon | 110 |
| | 995 |

Setzt man obiger Base 15 Gewichtsteile der neuen Verbindung Ib zu, so wird die Citrus-Bergamotte-Note sehr vorteilhaft unterstrichen. Die Galbanum-Note wirkt nun «angenehm eingekleidet».

Beispiel 17
Parfümerie-Komposition mit allgemein blumiger Note

| | Gewichtsteile |
|---|---|
| Dipropylenglykol | 200 |
| Limonen | 150 |
| α-Jonon | 60 |
| Citronellol | 50 |
| Linalool | 50 |
| Vertofix® | 50 |
| Galaxolide 50® | 50 |
| Benzylacetat | 30 |
| Myrascone® Giv | 30 |
| Jasmin synth. | 20 |
| Keton-Moschus | 20 |
| Phenyläthyltiglat | 20 |
| Weihrauch odor. (50% in Propylenglykol) | 15 |
| Citronellylacetat | 10 |
| cis-3-Hexenylacetat (10% in Propylenglykol) | 10 |
| Ylangöl | 10 |
| Ylang synth. | 10 |

| | Gewichtsteile |
|---|---|
| Zitronenöl | 15 |
| 2,2,8-Trimethyl-7-nonen-3-ol | 15 |
| γ-Undecalacton | 5 |
| Cyclamenaldehyd | 5 |
| Galbanum | 5 |
| Sandelholzöl | 5 |
| Jonquille absolut (10% in Propylenglykol) | 5 |
| Ciste Labdanumöl | 5 |
| Adoxal (2,6,10-Trimethyl-9-undecen-1-al) (10% in Propylenkol) | 5 |
| | 830 |

Gibt man zu obiger blumigen Base 170 Teile der neuen Substanz Ia, entsteht ein ausgesprochen «kosmetischer Effekt» in der neuen Komposition, welch letztere nun gleichzeitig viel diffusiver, frischer und lieblicher wirkt.

Beispiel 18
Parfümerie-Base in Richtung Gardenia

| | Gewichtsteile |
|---|---|
| Hydroxycitronellal | 150 |
| Bergamotteöl | 140 |
| α-Jonen | 100 |
| α-Amylzimtaldehyd | 85 |
| Heliotropin | 80 |
| Styrallylacetat | 80 |
| Ylang-Ylang-Öl | 80 |
| Benzylacetat | 80 |
| Phenyläthylalkohol | 80 |
| Linalool | 80 |
| γ-Nonalacton (10% in Dipropylenglykol) | 20 |
| Jasmin synth. | 15 |
| γ-Undecalacton (10% in Dipropylenglykol) | 10 |
| | 1000 |

Der Zusatz von 15% des neuen Gemisches A zu obiger Grund-Komposition lässt deren Kopfnote wesentlich voller erscheinen. Man beobachtet eine eindeutig erhöhte Diffusion bei gleichzeitiger Änderung von spitzigem Gardenia zu einem leicht veilchenartigen, weicher-blumigen Geruch, der sich sehr gut für Kosmetika eignet.

Beispiel 19
Fruchtige Base

| | Gewichtsteile |
|---|---|
| Limonen | 500 |
| Linalool | 200 |
| cis-3-Hexenylbenzoat | 60 |
| Benzylacetat | 40 |
| Citronellol | 30 |
| 2,2,8-Trimethyl-7-nonen-3-ol | 30 |
| cis-3-Hexenylacetat (10% in DGP) | 20 |
| Phenyläthyltiglat | 20 |
| γ-Undecalacton | 10 |
| Citronellylacetat | 10 |
| | 920 |

Der Zusatz von 80 Gewichtsteilen des neuen Gemisches A zu obiger Komposition führt zu einer äusserst interessanten Bereicherung der Citrusfruchtnote; die neue Komposition wirkt weniger hart, ohne dass der Eigengeruch der neuen Substanz durchdringt.

**Patentansprüche**

1. Verbindungen der Formel

$R^3$ $R^2$ $R^1$ OH $CH_3$
| | | | |
$R^4$-CH-CH-CH-CH-C=CH-$CH_2$-$CH_3$ I

worin eines der Symbole $R^1$, $R^2$ und $R^3$ für Methyl oder Äthyl und die anderen für Wasserstoff stehen und $R^4$ Wasserstoff oder Methyl bedeutet, wobei im Falle $R^1$ = $R^2$ Wasserstoff und $R^3$ = Methyl, $R^4$ Wasserstoff darstellt.

2. Verbindungen der Formel

R OH $CH_3$
| | |
R-$CH_2$-$CH_2$-CH-$CH_2$-CH-C=CH-$CH_2$-$CH_3$ I'

worin das eine Symbol R für Methyl und das andere für Wasserstoff steht.

3. 3,6-Dimethyl-6-nonen-5-ol.
4. 4-Methyl-3-decen-5-ol.
5. 4,6-Dimethyl-3-nonen-5-ol.
6. 4-Methyl-3-nonen-5-ol.
7. 4-Methyl-6-äthyl-3-octen-5-ol.
8. 3,6-Dimethyl-6-nonen-5-ol bzw. 4-Methyl-3-decen-5-ol im Gemisch mit 2,6-Dimethyl-6-nonen-5-ol.
9. Eine Verbindung, ausgewählt aus 4,7-Dimethyl-3-octen-5-ol, 4,6-Dimethyl-3-octen-5-ol, 4-Methyl-6-äthyl-3-nonen-5-ol, 4-Methyl-7-äthyl-3-nonen-5-ol, 4,8-Dimethyl-3-decen-5-ol.
10. Riech- und/oder Geschmackstoffkomposition, gekennzeichnet durch einen Gehalt an einer Verbindung der Formel

$R^3$ $R^2$ $R^1$ OH $CH_3$
| | | | |
$R^4$-CH-CH-CH-CH-C=CH-$CH_2$-$CH_3$ I

worin eines der Symbole $R^1$, $R^2$ und $R^3$ für Methyl oder Äthyl und die anderen für Wasserstoff stehen und $R^4$ Wasserstoff oder Methyl bedeutet, wobei im Falle $R^1$ = $R^2$ = Wasserstoff und $R^3$ = Methyl, $R^4$ Wasserstoff darstellt.

11. Riech- und/oder Geschmackstoffkomposition, gekennzeichnet durch einen Gehalt an einer Verbindung der Formel

R OH $CH_3$
| | |
R-$CH_2$-$CH_2$-CH-$CH_2$-CH-C=CH-$CH_2$-$CH_3$ I'

worin das eine Symbol R für Methyl und das andere für Wasserstoff steht.

12. Komposition gemäss Anspruch 11, dadurch gekennzeichnet, dass sie 3,6-Dimethyl-6-nonen-5-ol und/oder 4-Methyl-3-decen-5-ol und 2,6-Dimethyl-6-nonen-5-ol enthält.

13. Verfahren zur Herstellung von Verbindungen der Formel

```
R³  R²  R¹  OH CH₃
|   |   |   |  |
R⁴-CH-CH-CH-CH-C=CH-CH₂-CH₃        I
```

worin eines der Symbole $R^1$, $R^2$ und $R^3$ für Methyl oder Äthyl und die anderen für Wasserstoff stehen und $R^4$ Wasserstoff oder Methyl bedeutet, wobei im Falle $R^1 = R^2 =$ Wasserstoff und $R^3 =$ Methyl, $R^4$ Wasserstoff darstellt, dadurch gekennzeichnet, dass man 2-Methyl-2-pentenal mit einer Verbindung der Formel

```
R³  R²  R¹
|   |   |
R⁴-CH-CH-CH-MgX        II
```

worin $R^1$ bis $R^4$ obige Bedeutung besitzen und X für Halogen steht, umsetzt.

14. Verwendung von Verbindungen der Formel

```
R³  R²  R¹  OH CH₃
|   |   |   |  |
R⁴-CH-CH-CH-CH-C=CH-CH₂-CH₃        I
```

worin eines der Symbole $R^1$, $R^2$ und $R^3$ für Methyl oder Äthyl und die anderen für Wasserstoff stehen und $R^4$ Wasserstoff oder Methyl bedeutet, wobei im Falle $R^1 = R^2 =$ Wasserstoff und $R^3 =$ Methyl, $R^4$ Wasserstoff darstellt, als Riech- und/oder Geschmackstoffe.

15. Verwendung von Verbindungen der Formel

```
            R           OH CH₃
            |           |  |
R-CH₂-CH₂-CH-CH₂-CH-C=CH-CH₂-CH₃        I'
```

worin das eine Symbol R für Methyl und das andere für Wasserstoff steht, als Riech- und/oder Geschmackstoffe.

16. Verwendung von 3,6-Dimethyl-6-nonen-5-ol und/oder 4-Methyl-3-decen-5-ol im Gemisch mit 2,6-Dimethyl-6-nonen-5-ol als Riech- und/oder Geschmackstoffe.

**Claims**

1. Compounds of the formula

```
R³  R²  R¹  OH CH₃
|   |   |   |  |
R⁴-CH-CH-CH-CH-C=CH-CH₂-CH₃        I
```

wherein one of the symbols $R^1$, $R^2$ and $R^3$ stands for methyl or ethyl and the others stand for hydrogen and $R^4$ signifies hydrogen or methyl, whereby $R^4$ represents hydrogen when $R^1 = R^2 =$ hydrogen and $R^3 =$ methyl.

2. Compounds of the formula

```
            R           OH CH₃
            |           |  |
R-CH₂-CH₂-CH-CH₂-CH-C=CH-CH₂-CH₃        I'
```

wherein the one symbol R stands for methyl and the other stands for hydrogen.

3. 3,6-Dimethyl-6-nonen-5-ol.
4. 4-Methyl-3-decen-5-ol.
5. 4,6-Dimethyl-3-nonen-5-ol.
6. 4-Methyl-3-nonen-5-ol.
7. 4-Methyl-6-ethyl-3-octen-5-ol.
8. 3,6-Dimethyl-6-nonen-5-ol or 4-methyl-3-decen-5-ol in admixture with 2,6-dimethyl-6-nonen-5-ol.
9. A compound selected from 4,7-dimethyl-3-octen-5-ol, 4,6-dimethyl-3-octen-5-ol, 4-methyl-6-ethyl-3-nonen-5-ol, 4-methyl-7-ethyl-3-nonen-5-ol, 4,8-dimethyl-3-decen-5-ol.
10. Odorant and/or flavouring substance composition, characterized by a content of a compound of the formula

```
R³  R²  R¹  OH CH₃
|   |   |   |  |
R⁴-CH-CH-CH-CH-C=CH-CH₂-CH₃        I
```

wherein one of the symbols $R^1$, $R^2$ and $R^3$ stands for methyl or ethyl and the others stand for hydrogen and $R^4$ signifies hydrogen or methyl, whereby $R^4$ represents hydrogen when $R^1 = R^2 =$ hydrogen and $R^3 =$ methyl.

11. Odorant and/or flavouring substance composition, characterized by a content of a compound of the formula

```
            R           OH CH₃
            |           |  |
R-CH₂-CH₂-CH-CH₂-CH-C=CH-CH₂-CH₃        I'
```

wherein the one symbol R stands for methyl and the other stands for hydrogen.

12. Composition according to claim 11, characterized in that it contains 3,6-dimethyl-6-nonen-5-ol and/or 4-methyl-3-decen-5-ol and 2,6-dimethyl-6-nonen-5-ol.

13. Process for the manufacture of compounds of the formula

```
R³  R²  R¹  OH CH₃
|   |   |   |  |
R⁴-CH-CH-CH-CH-C=CH-CH₂-CH₃        I
```

wherein one of the symbols $R^1$, $R^2$ and $R^3$ stands for methyl or ethyl and the others stand for hydrogen and $R^4$ signifies hydrogen or methyl, whereby $R^4$ represents hydrogen when $R^1 = R^2 =$ hydrogen and $R^3 =$ methyl, characterized by reacting 2-methyl-2-pentenal with a compound of the formula

```
R³  R²  R¹
|   |   |
R⁴-CH-CH-CH-MgX        II
```

wherein $R^1$ to $R^4$ have the above significance and X stands for halogen.

14. Use of compounds of the formula

```
R³  R²  R¹  OH CH₃
|   |   |   |  |
R⁴-CH-CH-CH-CH-C=CH-CH₂-CH₃        I
```

wherein one of the symbols $R^1$, $R^2$ and $R^3$ stands for methyl or ethyl and the others stand for hydrogen and $R^4$ signifies hydrogen or methyl, whereby $R^4$ represents hydrogen when $R^1 = R^2 =$ hydrogen and $R^3 =$ methyl, as adorant and/or flavouring substances.

15. Use of compounds of the formula

```
          R          OH CH3
          |          |  |
R-CH2-CH2-CH-CH2-CH-C=CH-CH2-CH3      I'
```

wherein the one symbol R stands for methyl and the other stands for hydrogen, as adorant and/or flavouring substances.

16. Use of 3,6-dimethyl-6-nonen-5-ol and/or 4-methyl-3-decen-5-ol in admixture with 2,6-dimethyl-6-nonen-5-ol as odorant and/or flavouring substances.

**Revendications**

1. Composés de formule:

```
   R3  R2  R1  OH CH3
   |   |   |   |  |
R4-CH-CH-CH-CH-C=CH-CH2-CH3       I
```

dans laquelle l'un des symboles $R^1$, $R^2$ et $R^3$ représente un groupe méthyle ou éthyle et les autres l'hydrogène, et $R^4$ représente l'hydrogène ou un groupe méthyle, étant spécifié que lorsque $R^1$ = $R^2$ = hydrogène et $R^3$ = méthyle, $R^4$ représente l'hydrogène.

2. Composés de formule:

```
          R          OH CH3
          |          |  |
R-CH2-CH2-CH-CH2-CH-C=CH-CH2-CH3      I'
```

dans laquelle l'un des symboles R représente un groupe méthyle et l'autre l'hydrogène.

3. Le 3,6-diméthyl-6-nonène-5-ol.
4. Le 4-méthyl-3-décène-5-ol.
5. Le 4,6-diméthyl-3-nonène-5-ol.
6. Le 4-méthyl-3-nonène-5-ol.
7. Le 4-méthyl-6-éthyl-3-octène-5-ol.
8. Le 3,6-diméthyl-6-nonène-5-ol ou le 4-méthyl-3-décène-5-ol en mélange avec le 2,6-diméthyl-6-nonène-5-ol.
9. Composé choisi parmi le 4,7-diméthyl-3-octène-5-ol, le 4,6-diméthyl-3-octène-5-ol, le 4-méthyl-6-éthyl-3-nonène-5-ol, le 4-méthyl-7-éthyl-3-nonène-5-ol, le 4,8-diméthyl-3-décène-5-ol.
10. Composition de parfum et/ou d'arôme, caractérisée en ce qu'elle contient un composé de formule:

```
   R3  R2  R1  OH CH3
   |   |   |   |  |
R4-CH-CH-CH-CH-C=CH-CH2-CH3       I
```

dans laquelle l'un des symboles $R^1$, $R^2$ et $R^3$ représente un groupe méthyle ou éthyle et les autres l'hydrogène, et $R^4$ représente l'hydrogène ou un groupe méthyle, étant spécifié que lorsque $R^1$ = $R^2$ = hydrogène et $R^3$ = méthyle, $R^4$ représente l'hydrogène.

11. Composition de parfum et/ou d'arôme, caractérisée en ce qu'elle contient un composé de formule:

```
          R          OH CH3
          |          |  |
R-CH2-CH2-CH-CH2-CH-C=CH-CH2-CH3      I'
```

dans laquelle l'un des symboles R représente un groupe méthyle et l'autre l'hydrogène.

12. Composition selon la revendication 11, caractérisée en ce qu'elle contient du 3,6-diméthyl-6-nonène-5-ol et/ou du 4-méthyl-3-décène-5-ol et du 2,6-diméthyl-6-nonène-5-ol.

13. Procédé de préparation des composés de formule:

```
   R3  R2  R1  OH CH3
   |   |   |   |  |
R4-CH-CH-CH-CH-C=CH-CH2-CH3       I
```

dans laquelle l'un des symboles $R^1$, $R^2$ et $R^3$ représente un groupe méthyle ou éthyle et les autres l'hydrogène, et $R^4$ représente l'hydrogène ou un groupe méthyle, étant spécifié que lorsque $R^1$ = $R^2$ = hydrogène et $R^3$ = méthyle, $R^4$ représente l'hydrogène, caractérisé en ce que l'on fait réagir le 2-méthyl-2-penténal avec un composé de formule:

```
   R3  R2  R1
   |   |   |
R4-CH-CH-CH-MgX        II
```

dans laquelle les symboles $R^1$ à $R^4$ ont les significations indiquées ci-dessus, et X représente un halogène.

14. Utilisation des composés de formule:

```
   R3  R2  R1  OH CH3
   |   |   |   |  |
R4-CH-CH-CH-CH-C=CH-CH2-CH3       I
```

dans laquelle l'un des symboles $R^1$, $R^2$ et $R^3$ représente un groupe méthyle ou éthyle et les autres l'hydrogène, et $R^4$ représente l'hydrogène ou un groupe méthyle, étant spécifié que lorsque $R^1$ = $R^2$ = hydrogène et $R^3$ = méthyle, $R^4$ représente l'hydrogène, en tant que substances odorantes et/ou aromatisantes.

15. Utilisation des composés de formule:

```
          R          OH CH3
          |          |  |
R-CH2-CH2-CH-CH2-CH-C=CH-CH2-CH3      I'
```

dans laquelle l'un des symboles R représente un groupe méthyle et l'autre l'hydrogène, en tant que substances odorantes et/ou aromatisantes.

16. Utilisation du 3,6-diméthyl-6-nonène-5-ol et/ou du 4-méthyl-3-décène-5-ol en mélange avec le 2,6-diméthyl-6-nonène-5-ol en tant que substances odorantes et/ou aromatisantes.